(19) Europäisches Patentamt
European Patent Office
Office européen des brevets

(11) EP 1 117 392 B1

(12) **EUROPEAN PATENT SPECIFICATION**

(45) Date of publication and mention of the grant of the patent:
**25.02.2004 Bulletin 2004/09**

(21) Application number: **99933763.7**

(22) Date of filing: **08.07.1999**

(51) Int Cl.[7]: **A61K 31/215**, A61K 31/40, A61K 31/19, A61K 31/225, A61P 9/10

(86) International application number:
**PCT/US1999/015385**

(87) International publication number:
**WO 2000/018395 (06.04.2000 Gazette 2000/14)**

(54) **METHOD FOR PREVENTING OR DELAYING CATHETER-BASED REVASCULARIZATION**

METHODE ZUR VERMEIDUNG ODER VERZÖGERUNG VON KATHETERBEDINGTER GEFÄSSRÜCKBILDUNG

PROCEDE POUR EMPECHER OU RETARDER LA REVASCULARISATION PAR CATHETER

(84) Designated Contracting States:
**AT BE CH CY DE DK ES FI FR GB GR IE IT LI LU MC NL PT SE**
Designated Extension States:
**AL LT LV MK RO SI**

(30) Priority: **30.09.1998 US 102457 P**

(43) Date of publication of application:
**25.07.2001 Bulletin 2001/30**

(73) Proprietor: **WARNER-LAMBERT COMPANY LLC**
**Morris Plains, New Jersey 07950 (US)**

(72) Inventor: **BLACK, Donald, Michael**
**Ann Arbor, MI 48105 (US)**

(74) Representative: **Mansmann, Ivo et al**
**Warner-Lambert Company,**
**Patent Department,**
**c/o Gödecke GmbH**
**79090 Freiburg (DE)**

(56) References cited:
**WO-A-95/13063**
**WO-A-97/16184**
**WO-A-97/35576**
**WO-A-98/01100**
**WO-A-99/30704**
**US-A- 5 648 387**

- **MCCORMICK LISA S., ET AL.: "Rationale, design, and baseline characteristics of a trial comparing aggressive lipid lowering with atorvastatin versus revascularization treatments" AM. JOUR. OF CARDIOL., vol. 80, no. 9, 1 November 1997 (1997-11-01), pages 1130-1133, XP002120664**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 117 392 B1

## Description

BACKGROUND OF THE INVENTION

Catheter-based revascularization procedures (such as percutaneous transluminal coronary angioplasty [PTCA], atherectomy, stents, and laser ablation) are intended to provide rapid and sustained pain relief to patients with symptomatic (angina pectoris) coronary artery disease (CAD). The effectiveness of PTCA, the most common of these procedures, in relieving angina and improving exercise tolerance is well-established. However, this method is limited by the risk of acute complications arising from the procedure, such as death, nonfatal myocardial infarction (MI), or need for emergency coronary artery bypass grafting (CABG). In addition, PTCA has technical limitations depending on the location of the stenosis, as well as a restenosis rate of up to 40%. PTCA and similar techniques are most successful when dilating short, limited sections of the coronary artery system. Stenoses greater than 2 cm in length are associated with decreased success rates. Other recanalization methods are being evaluated to attempt to circumvent some of the problems associated with PTCA, but these methods are burdened by similar rates of restenosis, and their own inherent disadvantages. No effective treatment for restenosis is known at this time.

Most importantly, the recanalization procedures do not influence the underlying disease which leads to the coronary artery stenoses, namely by formation of atherosclerotic plaques. There are several potential mechanisms by which lipid-lowering therapy may affect the disease process.

The first of these is the observation from many studies that by reducing low-density lipoprotein cholesterol (LDL-C), one can slow the progression of atherosclerosis, or even induce regression of existing plaques. However, the slight increase in lumen area resulting from plaque regression may not be sufficient to explain the substantial accompanying reduction in coronary ischemic events.

A second hypothesis is that lipid-lowering therapy may induce soft, fatty plaques to become more fibrous, thereby reducing the risk to rupture suddenly. It has been observed that the majority of clinical events are caused by mild to moderate lesions (<70% stenosis) abruptly progressing to severe obstruction. Histologic findings suggest that a large lipid pool, and profuse lipid-laden foam cells in the fibrous cap of the atheroma, predispose it to fissuring and subsequent plaque disruption. Experimental and mechanical studies have demonstrated that lipid-lowering therapy decreases the number of lipid-laden intimal macrophages and, early in the regression process, hydrolyze liquid cholesteryl ester to crystalline cholesterol monohydrate. This may increase the "stiffness" of the lipid pool. Lipid-lowering therapy eventually depletes both core cholesteryl ester and cholesterol monohydrate deposits. Thus, lipid-lowering therapy may act to stabilize lesions, in addition to reducing their size. However, the correlation between lipid stiffness and a reduction in cardiac events is still speculative.

A third theory is that decreasing plasma cholesterol leads to an improvement in endothelial dysfunction. Both human and animal studies have demonstrated that CAD and/or hypercholesterolemia impairs the coronary arterial vasodilation mediated by the endothelium. Animal studies have demonstrated improved endothelium-dependent vasodilation after cholesterol lowering. A study by Gould, et al, in 15 patients suggests that intensive cholesterol lowering over a period of 90 days improves myocardial perfusion capacity in patients with CAD, as evidenced by positron emission tomography (PET) after intravenous dipyridamole, most likely through an improvement in endothelial function.

Last, a number of hemorrheological factors (eg, fibrinogen, Factor VII, plasma viscosity, hematocrit, red blood cell aggregation, and total white cell count) may contribute to both acute thrombotic events, and to the development of atherosclerosis. Lipid-lowering therapy has been shown to improve whole blood viscosity, plasma viscosity, and red cell aggregation.

Irrespective of the actual mechanism, many clinical trials have demonstrated a correlation between lipid lowering and improved cardiac outcomes. A recently published study, the Scandinavian Simvastatin Survival Study, also demonstrated that lipid lowering, in a hyperlipidemic population, led to a 42% reduction in cardiac mortality, and more than a 30% reduction in total mortality.

Although hypercholesterolemia is one of the most important risk factors for CAD, no studies have compared the long-term outcome of aggressive lipid lowering to recanalization of the coronary arteries. One study that compared another mechanism of medicinal treatment to surgery was the Angioplasty Compared to Medicine (ACME) study. The ACME study compared the effects of PTCA with medical therapy (antianginal medication) on angina and exercise tolerance in patients with single-vessel CAD. Although PTCA offered earlier and more complete relief of angina than medical therapy, and was associated with better performance on the exercise test, the PTCA treatment group had a higher incidence of cardiac events and repeat revascularizations.

Although PTCA is sometimes used in asymptomatic patients, or those with only mild symptoms of angina pectoris despite the presence of a positive exercise tolerance test, it is debatable whether PTCA or similar interventions are indicated in this patient population.

I have now established, in an open-label, randomized, multicenter study in asymptomatic or mildly to moderately symptomatic patients with LDL-C ≥130 mg/dL, that there is a dramatic improvement in clinical outcomes between

aggressive lipid lowering with atorvastatin compared to a recanalization procedure. Aggressive lipid lowering with atorvastatin significantly delays or even avoids the need for recanalization in these patients.

Atorvastatin is an HMG-CoA reductase inhibitor currently marketed as Lipitor®. It is a member of the "statin" class of organic compounds. Preclinical and clinical studies have demonstrated that atorvastatin reduces plasma total cholesterol, LDL-C, very low-density lipoprotein cholesterol (VLDL-C), and triglycerides (TG). Additionally, clinical studies have shown that atorvastatin appears to have several potential advantages over currently marketed statin reductase inhibitors, eg, enhanced LDL-C and TG lowering. These factors may augment its potential effect on cardiac outcomes.

In a 6-week, placebo-controlled, dose-ranging study in 79 hypercholesterolemic patients, doses of atorvastatin from 2.5 to 80 mg/day reduced LDL-C from 25% to 61%, significantly reduced total cholesterol and apolipoprotein B (apo B), and reduced TG levels up to 32%. No significant changes in high-density lipoprotein cholesterol (HDL-C), apolipoprotein AI (apo AI) or Lp (a), and no drug-related serious adverse events were reported. Other Phase 2 and 3 clinical studies in over 400 subjects and patients have supported these results. The most frequently occurring adverse events for patients were headache, infection (mostly colds), constipation, and back pain.

Although not yet studied in humans, a study in rabbits shows atorvastatin has at least as potent an effect on atherosclerotic lesions as other reductase inhibitors. Treatment of cholesterol-fed male New Zealand White rabbits with equi-efficacious doses of different HMG-CoA reductase inhibitors for 8 weeks resulted in specific changes in lesion size, monocyte-macrophage area, and the relative cellular composition of the atherosclerotic lesions. Atorvastatin reduced the size of the iliac-femoral lesions by approximately 68%, and significantly reduced the extracellular matrix and smooth muscle cell area by 63%, and reduced the percentage of discernible thoracic aortic lesions compared to progression controls. Atorvastatin caused these effects to a greater extent than the other statin reductase inhibitors evaluated.

## SUMMARY OF THE INVENTION

This invention provides the use of a cholesterol lowering agent for preventing or delaying catheter-based revascularization in patients suffering from coronary artery disease and in need of such treatment whereby said cholesterol lowering agent is to be administered in an amount to cause an aggressive lipid lowering. Hereby a cholesterol lowering agent selected from the statin class of HMG-CoA reductase inhibitors for example atorvastatin, pravastatin, simvastatin, fluvastatin, and mevastatin is to be administered. Other cholesterol lowering agents to be administered include those selected from the fibrate class, for example gemfibrozil, ciprofibrate, and bezafibrate. In another embodiment, a carboxyalkyl ether cholesterol lowering agent is used to aggressively lower LDL cholesterol according to this invention, and thereby prevent or delay the need for catheter-based revascularization in a patient suffering from coronary artery disease.

## DETAILED DESCRIPTION OF THE INVENTION

The compounds to be used in this invention are those which are effective at lowering cholesterol (LDL) in animals. Typical of the commonly used cholesterol lowering agents are the fibrates, the HMG-CoA reductase inhibitors, ie, the statins, and a new group of compounds known as the carboxyalkyl ethers. The cholesterol lowering agents are to be administered at a dose and frequency so as to aggressively lower the LDL cholesterol levels. The term "aggressively lowering LDL cholesterol levels" means reducing the serum LDL cholesterol by at least forty percent from baseline, and preferably by about fifty percent or more. This level of LDL cholesterol generally corresponds to an LDL serum concentration of about 100 mg/dL or lower, preferably about 80mg/dL, and even lower to about 70mg/dL or lower. To achieve aggressive lowering of cholesterol, the cholesterol lowering agent will generally be administered at a dose sufficient to effect such lowering of LDL-C below about 100 mg/dL. For example, a typical dose of atorvastatin utilized to achieve aggressive cholesterol lowering is about 50 to about 150 mg per day, preferably about 80 mg per day, for an adult of normal weight of about 70 kg. Other cholesterol lowering agents can similarly be administered at doses to effect aggressive cholesterol lowering, and in some cases the agents may be used in combination with other agents in order to achieve such aggressive cholesterol lowering according to this invention.

Numerous cholesterol lowering agents are known and can be used to cause aggressive cholesterol lowering according to the method of this invention. Typical agents to be used in the method of this invention include, but are not limited to, fibrates (eg, clofibrate, gemfibrozil, fenofibrate, ciprofibrate, and bezafibrate), niacin, carboxyalkylethers, thiazolinediones, eicosapentaenoic acid (EPA) and EPA-containing compositions (eg, Max EPA, SuperEPA).

Thiazolinediones useful in the present invention include, for example, darglitazone, Pioglitazone, BRL49653 (rosiglitazone), and troglitazone.

Carboxyalkylethers useful in the invention are described in U.S. Patent No. 5,648,387. Specifically, such compounds have the structure of Formula I

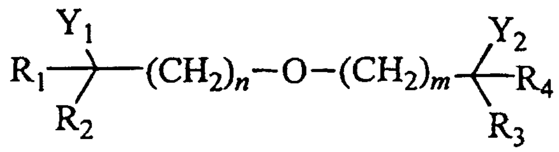

I

wherein

n and m independently are integers from 2 to 9;
$R_1$, $R_2$, $R_3$, and $R_4$ independently are $C_1$-$C_6$ alkyl, $C_2$-$C_6$ alkenyl, $C_2$-$C_6$ alkynyl, and $R_1$ and $R_2$ together with the carbon to which they are attached, and $R_3$ and $R_4$ together with the carbon to which they are attached, can complete a carbocyclic ring having from 3 to 6 carbons;
$Y_1$ and $Y_2$ independently are COOH, CHO, tetrazole, and $COOR_5$ where $R_5$ is $C_1$-$C_6$ alkyl, $C_2$-$C_6$ alkenyl, $C_2$-$C_6$ alkynyl;

and where the alkyl, alkenyl, and alkynyl groups may be substituted with one or two groups selected from halo, hydroxy, $C_1$-$C_6$ alkoxy, and phenyl.

Preferred carboxyalkylethers for use in the invention have the above formula wherein n and m are the same integer, and wherein $R_1$, $R_2$, $R_3$, and $R_4$ each are alkyl.

Further preferred carboxyalkylethers are those in which $Y_1$ and $Y_2$ independently are COOH or $COOR_5$ where $R_5$ is alkyl.

The most preferred carboxyalkylethers for use in the invention have the formula

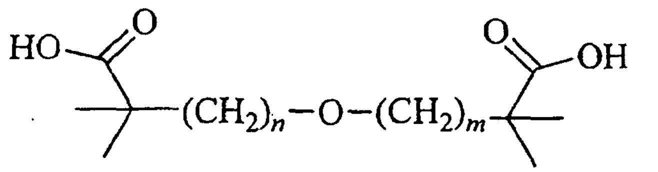

wherein n and m are each an integer selected from 2, 3, 4, or 5, ideally 4 or 5.

An especially preferred carboxyalkylether for use in the invention has the formula

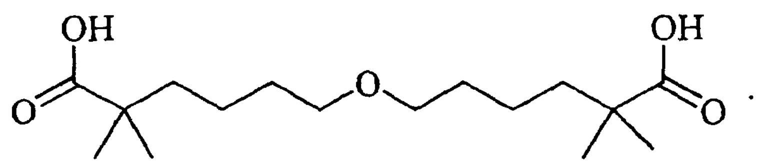

The ability of cholesterol lowering agents to cause an aggressive lowering of LDL cholesterol and to thereby prevent or delay the need for catheter-based revascularization in patients diagnosed with coronary artery disease has been established in clinical trial studies. The following detailed examples are provided by way of illustrating the significant ability of aggressive cholesterol lowering according to this invention to prevent or delay the need for catheter-based revascularization.

EXAMPLE 1

Atorvastatin was evaluated in a multi-center, open-label, clinical trial involving 341 patients. This trial is intended to determine whether aggressive lipid lowering with a cholesterol lowering agent can significantly delay or even obviate the need for catheter-based revascularization in patients diagnosed with coronary artery disease.

**STUDY OBJECTIVE(S)**

The objective of this study is to compare the clinical courses of aggressive lipid lowering with atorvastatin

versus revascularization treatments (AVERT) followed by usual care in asymptomatic or mildly to moderately symptomatic patients with CAD and LDL-C ≥130 mg/dL who are referred for a recanalization procedure. This will be determined by evaluating cardiovascular outcomes, all-cause mortality, quality of life, an economic assessment of outcomes, and safety measures.

**SUMMARY OF STUDY**

This is an 18-month, open-label, randomized, multicenter study in 341 patients with elevated LDL-C (≥130 mg/dL whether or not they are receiving lipid-lowering medication) who have had angiography. Patients who may qualify for the study are those whose angiogram indicates that they are eligible for a recanalization procedure and who have asymptomatic or mildly to moderately symptomatic CAD (with regard to angina pectoris symptoms with or without antianginal therapy). Patients must be Class I or II based on the Canadian Cardiovascular Society classification of angina severity, and must be able to complete 4 minutes of a Bruce protocol treadmill test, or a 20-W/min supine bicycle exercise test. Qualified patients will be randomized to one of 2 treatment groups: Group 1 will receive atorvastatin 80 mg QD, and Group 2 will undergo a recanalization procedure followed by usual care.

**STUDY POPULATION**

**Source and Number of Patients**

**Source:** Patients will be recruited from the population of patients who have just undergone or are about to undergo angiography and in whom a recanalization procedure is recommended.

**Number of Patients:** 341 (163 patients received atorvastatin, 178 received revascularization)

**Patient-Selection Criteria**

*Inclusion Criteria*

- Men or women. Women of childbearing potential may be enrolled but must plan not to become pregnant during the course of this study and must practice a method of birth control that is considered suitable by the investigator. If established on hormonal contraceptives for more than 3 months, patients will be allowed to participate, providing this therapy remains constant throughout the study. If a patient becomes pregnant or begins breast-feeding during the study and is receiving atorvastatin, she must be withdrawn immediately;
- Patients in whom a regulatory agency-approved recanalization procedure is recommended;
- Patients with ≥1 major coronary vessel never subjected to intervention with ≥50% stenosis;
- Patients with LDL-C ≥130 mg/dL (≥3.4 mmol/L) as calculated by the Friedewald formula;
- Patients are allowed to be taking lipid-lowering medication at the time the LDL-C is determined;
- Patients with serum TG ≤400 mg/dL (≤4.5 mmol/L);
- Patients who can complete 4 minutes of a Bruce protocol treadmill test or a 20-W/min supine bicycle test without developing objective evidence of ischemia (ST segment depression of ≥2 mm); and,
- Patients with asymptomatic or mildly to moderately symptomatic CAD (Class I or II of the Canadian Cardiovascular Society (CCS) classification of angina severity.

*Exclusion Criteria*

- Patients under 18 or over 80 years of age;
- Women who are breast-feeding or who are pregnant;
- Patients with left main disease, or 3-vessel disease (defined as presence of >50% stenosis in all 3 major coronary arteries);
- Patients with unstable angina or patients who experience angina after mild exercise;
- Patients who have been recommended to have a direct immediate angioplasty for evolving acute MI;
- Patients who have experienced an MI within the previous 28 days;
- Patients with known ejection fraction <40%;
- Patients with CHF who are NYHA Class III or IV;
- Patients who have undergone a CABG, unless current recanalization is recommended for a native vessel and current angiogram indicates bypass grafts are present;
- Patients in whom a CABG is recommended based on the current angiogram;
- Patients who underwent a recanalization procedure during the previous 6 months;
- Patients consuming an average of more than 21 alcoholic drinks per week (approximately 277 g of pure alcohol);
- Patients with significant renal dysfunction, active liver disease, or hepatic dysfunction, SGOT (AST) or SGPT (ALT) >2 × the upper limit of normal (ULN) range;
- Patients with CPK levels >3 × ULN or unexplained elevations of CPK;
- Patients having uncontrolled hypertension, defined as a diastolic blood pressure >100 mm Hg identified as the disappearance of all sound (Korotkoff Phase V) after sitting quietly for at least 3 minutes, whether taking or not taking a concurrent antihypertensive medication;
- Patients who may be unreliable as study participants based on the investigator's prior knowledge of the patient, such as someone who abuses alcohol;
- Patients participating in another clinical study concurrently or within the 30-day phase prior to screening for entry into the present study;
- Patients with known hypersensitivity to HMG-CoA-reductase inhibitors;
- Patients with other significant abnormalities that the investigator feels may compromise the patient's safety or successful participation in the study.

**Prohibited Medications or Precautions**

The following medications are not allowed in Group 1 (atorvastatin therapy) during this study:

- Lipid-regulating drugs: niacin, probucol, Metamucil® (>2 tablespoons per day), fibrates and derivatives, other HMG-CoA reductase inhibitors, or fish oils;
- Immunosuppressive agents; and,

- Drugs known to be associated with rhabdomyolysis in combination with HMG-CoA-reductase inhibitors (eg, cyclosporine, erythromycin, gemfibrozil, or niacin).

There are no study restrictions on medications for patients in Group 2 (recanalization procedure followed by usual care).

The dosage and regimen of any chronic, permitted concurrent medication should be managed appropriately throughout the study.

Patients in Group 1 who were receiving a lipid-lowering medication during the screening period must discontinue such medication at the time of randomization and begin atorvastatin instead. Patients in Group 2 who were receiving lipid-lowering medication during screening may continue on this medication, if desired.

The occasional use of antacids is permitted.

Intensive antianginal therapy is strongly recommended for patients in both treatment groups.

## STUDY DESIGN AND METHODOLOGY

### Study Design

This is an 18-month, open-label, randomized, parallel-group study comparing aggressive lipid lowering with atorvastatin (80 mg QD) to a recanalization procedure followed by usual care. The study is conducted in patients with LDL-C ≥130 mg/dL and TG ≤400 mg/dL who have asymptomatic or mildly to moderately symptomatic CAD (with or without antianginal therapy) and in whom a recanalization procedure is recommended. "Usual care" represents a heterogeneous mix of therapies that may include calcium channel blocker use or lipid-lowering therapy (including diet, behavior modification or antihyperlipidemic medication). Patients who have undergone angiography, or are about to undergo angiography, will be considered for this study based on the results of the angiogram and other inclusion criteria. The study will be performed using a common protocol at all study sites. The investigators and patients will not be blinded to treatment group, but will be blinded to lipid measurements throughout the study.

### Study Schedule

The schedule of visits for this study and procedures to be performed at each visit are closely monitored. However, a patient may be seen at any time for reasons of safety. There should be no more than 6 weeks between the screening visit (Visit S1) and the first visit of the treatment phase (Visit T1), and no more than 8 weeks between the angiogram and the original recanalization procedure for patients in Group 2. All laboratory procedures, such as clinical laboratory measurements and lipoprotein and apolipoprotein determinations used for efficacy measurements, will be performed by a central laboratory. Part of the sample obtained at the screening visit may be sent to a local laboratory for a quick response to determine patient eligibility. The rest of the sample should be sent to the central laboratory (split sample).

The timing of clinic visits should be consistent throughout the study and, for patients in Group 1, atorvastatin should be taken at approximately the same time each day, ie, at bedtime. Clinic visits during all phases of the study at which lipid profiles will be determined must occur after a minimum 12-hour fast (water allowed). The only exception to this is the screening visit at which it is preferred, but not required, that patients be in a fasting state. For patients in Group 1, the blood sample must be drawn 6 to 18 hours after the previous dose of study medication. The foregoing study design is illustrated below in Table 1.

Table 1. Study Design

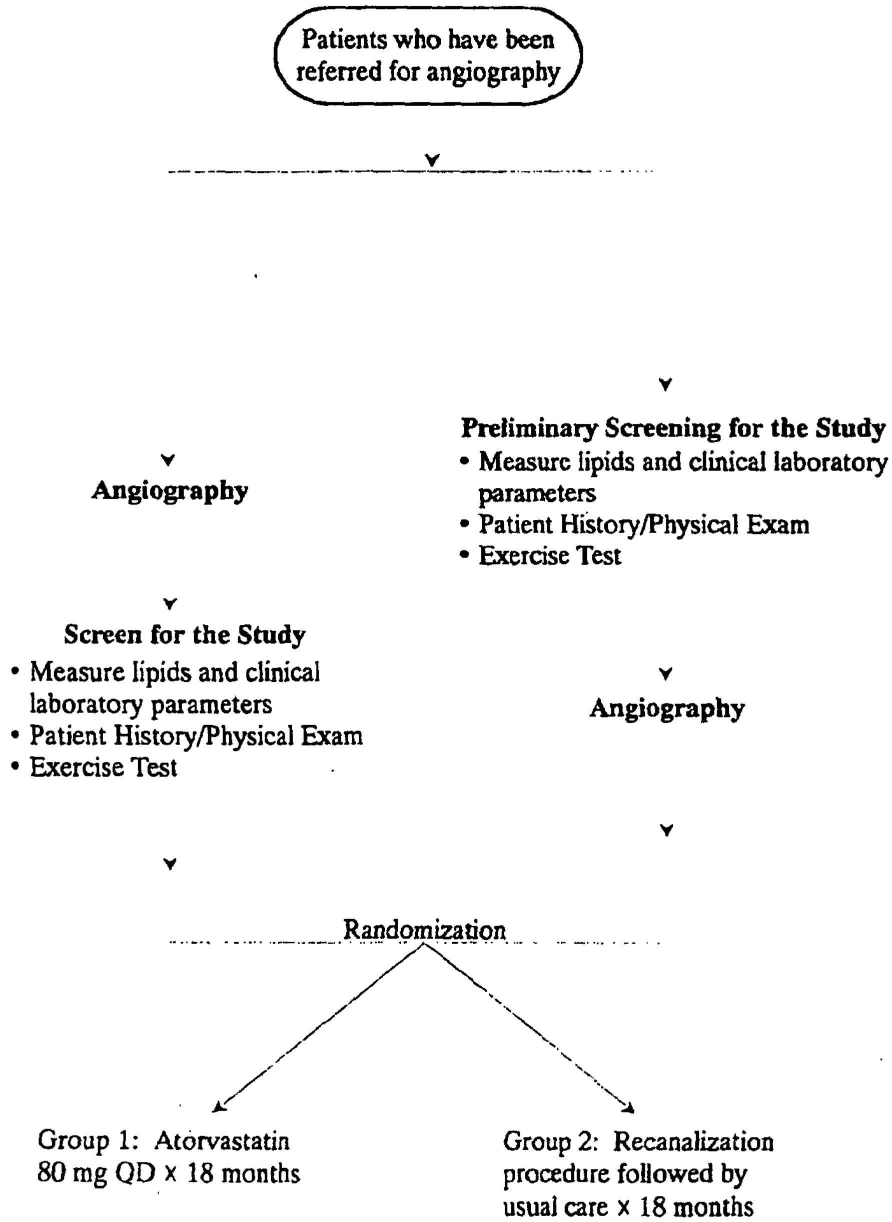


*Screening Phase*

The purpose of the screening phase is to assess a patient's qualification for randomization to the study.

If the institution commonly performs the angiography and then performs the recanalization procedure at a later date, the following sequence of events should be followed (per Table 1):

Patients who have undergone an angiography and who meet the coronary inclusion criteria and who have none of the coronary exclusion criteria will be evaluated for this study. The investigator should inform the patient about the study, obtain the patient's medical history and informed consent, draw blood for lipid measurements and clinical laboratory parameters, and

perform an exercise test and physical examination. If he or she qualifies for the study on the basis of his or her lipid measurements and exercise test results, and has none of the exclusion criteria, he or she will be randomized to receive atorvastatin, or to undergo the recanalization procedure followed by usual care, depending on the randomization code. All screening procedures must be completed and the patient randomized within 6 weeks of the angiogram. The original recanalization procedure in Group 2 must be completed within 8 weeks of the angiogram.

If the institution commonly performs the recanalization procedure during the catheterization for the angiogram, the following sequence of events should be followed:

The investigator should inform the patient about the possibility of qualifying for this study and should obtain informed consent before angiography. In addition, a medical history, exercise test, and physical exam should be completed, and blood should be drawn for lipid measurements and clinical laboratory parameters. If the patient qualifies for the study on the basis of his/her lipid measurements and exercise test results, and has none of the exclusion criteria, he or she should undergo angiography and then, if he or she meets the coronary artery inclusion criterion and has none of the coronary exclusion criteria, be randomized to either receive atorvastatin or undergo the recanalization procedure followed by usual care, depending on the randomization code.

Patients at North American sites must follow a Bruce protocol for a treadmill exercise test, while patients at European sites may follow either a Bruce protocol or a 20-W/min supine bicycle test. The protocols for either test are well-known. In order to qualify for the study, patients must be able to complete 4 minutes of the Bruce protocol or a 20-W/min supine bicycle test without developing objective evidence of ischemia (ST segment depression of ≥2 mm). If a patient is able to complete 4 minutes, he or she should complete as much of the remainder of the test as he or she is able.

When blood is drawn for screening clinical laboratory measurements, the sample should be split. Half of the sample should be sent to the local laboratory for a quick response to determine patient eligibility, and the other half should be sent to the central laboratory. All subsequent samples should be sent in their entirety to the central laboratory.

Results of the angiography will be recorded (retrospectively in some cases) in the study CRFs. The angiography films should be stored at the site as source documents.

Randomization must occur no later than 6 weeks after angiography and the intervention for patients in Group 2 must occur within 8 weeks of angiography.

*Open-Label Treatment Phase*

All patients will be randomized to either Group 1 or Group 2 according to a randomization code. Patients in Group 1 should discontinue any lipid-lowering medication they may have been receiving and start open-label therapy with atorvastatin 80 mg (two 40-mg tablets) taken once daily at bedtime. Patients in Group 2 will undergo the recommended recanalization procedure followed by usual care. "Usual care" represents a heterogenous mix of therapies that may or may not include lipid-lowering therapy (eg, diet, behavior modification, or antihyperlipidemic medication). Usual care will be determined by the investigator or the patient's primary physician (or whoever typically decides the patient's follow-up care) as he or she sees fit. These patients will return to the investigator as scheduled and report what therapy(ies) they are receiving. Since any of a number of people may be dictating a patients' follow-up care, and any of a number of people may be seeing the patient for study visits (eg, the investigator, a nurse, a fellow), we will provide a standard list of questions to ask these patients to try to reduce bias.

Patients in both treatment groups should take one aspirin daily. Optimal antianginal therapy is strongly recommended for all patients.

Visit T1 (Week 0) is the visit at which qualifying patients will be randomized. This will be the same day as the angiography and recanalization procedure (for patients in Group 2) at institutions that combine the angiography with the recanalization procedure. At institutions that separate the angiography from the recanalization procedure, it is the day that the investigator knows that the patient meets all of the inclusion criteria and none of the exclusion criteria, assigns the patient a number, and opens the randomization code to determine to which treatment group the patient is randomized. It will not necessarily be the day that the recanalization procedure will be performed for patients in Group 2.

For the purposes of this study, some information about the original recanalization procedure in Group 2, and any other recanalization procedures in either group, will be recorded in the study CRFs. This includes type of intervention, intervention vessel, the percent stenosis before and after the intervention, and the success of the procedure (defined as a >20% change in luminal diameter narrowing and a residual diameter <50% stenosis without the occurrence of death, acute MI, or need for acute CABG during hospitalization).

In order to have a revascularization procedure after randomization (and after the original recanalization procedure in Group 2) a patient should demonstrate objective evidence of a deterioration in his or her condition, defined as one of the following:

- Rest angina with ECG changes; or

- A 3-minute deterioration on a Bruce protocol treadmill test or supine bicycle test (time to ≥2 mm ST segment depression) while on maximal antianginal therapy; or
- An acute myocardial infarction verified by objective evidence.

Patients who experience a nonfatal ischemic event or who undergo a revascularization procedure (other than the initial recanalization in Group 2) at any time after randomization should not be dropped from the study.

Clinic visits will occur after 6 and 12 weeks, 6 months, and then every 6 months thereafter. At all visits, blood samples will be drawn and sent to the central laboratory for the determination of safety parameters, lipoproteins, and additional parameters. The central lab will notify the site and the sponsor if a patient's LDL-C decreases to <50 mg/dL. The investigator may choose to reduce the dose of atorvastatin at his or her discretion. A patient may be seen at any time for reasons of safety.

The quality-of-life questionnaire, the SF-36 Health Survey (SF-36), will be administered at baseline (Visit T1), at Month 6, and at the end of the study. This is a short form, 36-item, questionnaire that was designed to measure general health status from the patient's point of view. The SF-36 is available in a number of languages and can generally be completed by patients in 5 to 10 minutes. During the course of the study, the patient should be encouraged to maintain a healthy lifestyle, which may include modifications in dietary, smoking, or exercise habits.

**Efficacy Assessment**

*Primary Efficacy Parameter(s)*

The primary efficacy parameter will be the incidence rates associated with an ischemic event in each treatment group. In addition to obtaining the proportion associated with an ischemic event, we also have an interest in evaluating (by treatment group) the total number of these ischemic events and the distribution of each of the events that define an ischemic event. An ischemic event is defined as the occurrence of at least one of the following events: cardiac death, cardiac arrest, nonfatal MI, ischemic CVA, CABG, recanalization (other than the original recanalization procedure in Group 2), or unstable angina with objective evidence.

*Secondary Efficacy Parameters*

The secondary efficacy evaluation will include the following:

- Time from randomization to the occurrence of an ischemic event;
- Change from baseline in angina class (CCS);
- Percent change from baseline in LDL-C, HDL-C, total cholesterol, total TG, apo AI, apo B, and Lp(a);
- Quality of life (QOL) determined by SF-36 questionnaire; and,
- All-cause mortality rates.

Other parameters of interest include:

- Patients in the atorvastatin group (Group 1) who undergo a PTCA or other recanalization procedure will have their subsequent ischemic incidence rates compared with patients in Group 2 (all the patients in this group have a PTCA or similar procedure);
- An evaluation of the number of PTCAs or other recanalization procedures required in the atorvastatin group compared to the usual care group; and,
- A correlation analysis between the incidence rates of the ischemic events and the LDL-C and triglyceride measurements.

*Economic Assessment*

This study will assess the cost of treating asymptomatic or mildly to moderately symptomatic patients with either atorvastatin or a recanalization procedure followed by usual care. Costs to be considered will include the cost

of clinical end points, the cost of the treatment regimens (recanalization procedure or atorvastatin medication), and costs associated with serious attributable adverse events. Serious attributable adverse events are defined for Group 2 as pseudoaneurysm, groin hematoma, access-site bleeding, abrupt vessel closure, dissection, or reaction to contrast dye; and for Group 1 as hepatitis or rhabdomyolysis. Generalized costs for provider services for the above-mentioned events and interventions will be obtained from national databases. In addition, these costs may also be collected from some or all of the participating study sites.

**Safety Assessment**

HMG-CoA-reductase inhibitors have been associated with 2 important side effects: elevated liver transaminases and the occurrence of myopathy. Accordingly, at each predetermined visit, all patients must have AST, ALT, and CPK measured as commonly recommended. Additionally, patients in Group 2 should be monitored as appropriate for the agents used.

*Physical Examination*

A physical examination and medical history will be performed at the screening visit (S1), and a repeat physical examination will be performed every 6 months thereafter. Significant detrimental changes will be recorded as adverse events.

*Adverse Events*

Adverse events will be recorded at each clinic visit and up to 15 days following the cessation of treatment.

*Laboratory Evaluation*

Full clinical laboratory determinations including urinalysis by Ames Multistix® will be performed at Screening, Week 0 (Visit T1), Month 6, and at the end of the study. An abbreviated clinical laboratory determination of key safety parameters (liver enzymes and CPK) will be performed at all other visits for patients in both treatment groups. A verified clinically significant laboratory abnormality occurring during the study will be reported as an adverse event and followed until the abnormality has resolved or a satisfactory explanation has been obtained.

Elevations in laboratory parameters that may be associated with the medical therapy that constitutes "usual care" (Group 2) should be managed as considered appropriate by the investigator/primary care physician.

Elevations in CPK and liver transaminase levels that occur during therapy with atorvastatin (Group 1) should be managed as follows:

- If at any time during the study the patient's ALT or AST levels increase to >3 × ULN range, the patient should be scheduled for a repeat laboratory measurement within 1 week (±3 days). If the repeat value is still >3 × ULN range, the dose should be reduced by 50% (to 40 mg QD). Patients who continue in the study at the reduced dose should have ALT/AST levels reassessed within 1 more week (±3 days). If the repeat value is still >3 × ULN range, the dosage should be halved once again to 20 mg QD. The dosage may again be halved to 10 mg QD, if necessary, or the patient may discontinue atorvastatin but remain in the study.

- If at any time during the study, the patient's CPK level increases to >10 × ULN range, the patient should be scheduled for a repeat laboratory measurement within 1 week (±3 days). If the repeat value is still >10 × ULN range WITH muscle tenderness or weakness, the patient must be withdrawn from atorvastatin but may remain in the study.

- If the repeat value is still > 10 × ULN range WITHOUT muscle tenderness or weakness, the dose should be reduced by 50% (to 40 mg QD). Patients who continue in the study at the reduced dose should have their CPK level reassessed within 1 week (±3 days). If the repeat value is still >10 × ULN range without symptoms the dose should be halved again to 20 mg QD. The dose may again be halved to 10 mg QD, if necessary, or the patient may discontinue atorvastatin but remain in the study.

Patients should be advised to report promptly unexplained muscle pain, tenderness, or weakness, particularly if accompanied by fever or malaise.

**NOTE:** Once a patient has decreased the atorvastatin dose due to elevated liver enzymes or elevated CPK levels, that patient cannot be titrated back to the higher dose. Consideration should be given to temporarily withholding or discontinuing atorvastatin in any patient with a risk factor predisposing to the development of renal failure secondary to rhabdomyolysis, including severe acute infection; hypotension; major surgery; trauma; severe metabolic, endocrine, or electrolyte disorders; and uncontrolled seizures.

*Safety Profile*

At each visit, the following minimum safety data will be collected: pulse, blood pressure, and weight.

*Electrocardiogram*

A standard resting 12-lead electrocardiogram will be performed during the beginning of the treatment phase (Visit T1) and every 6 months thereafter.

### DATA ANALYSIS AND STATISTICAL CONSIDERATIONS

### Sample Size Considerations

The statistical analysis associated with this study considers a number of parameters. The primary efficacy parameter is the incidence rate of an ischemic event (defined as the occurrence of at least one of the following events: cardiac death, cardiac arrest, nonfatal MI, ischemic CVA, CABG, recanalization [other than the original procedure in Group 2], or unstable angina with objective evidence. The secondary parameter analysis considers: the time from randomization to the occurrence of an ischemic event; the change from baseline in angina class; the percent change from baseline to the end of treatment on a number of lipid parameters; a QOL measure (based on the results of the SF-36 Health Survey); and all-cause mortality rates.

The sample size is based on the number of patients required to obtain a reliable statistical test to test the difference between the 2 groups on incidence rates of ischemic events. In addition, the sample size recommended will provide an adequate sample size for a powerful statistical test to compare the 2 groups on time to an ischemic event and the change from baseline in lipid parameters.

Assuming an incidence rate of 35% for the usual care group and a 20% incidence rate for the atorvastatin group; the recommended sample size is 340 patients (170/group). The sample size derived provides a 0.05 level of significance with 85% power for detecting a significant difference between the 2 groups of these incidence rates. In deriving the sample size, we took into consideration an expected 5% patient drop-out rate.

The statistical analyses will be performed on all randomized patients.

### Analysis of the Primary Efficacy Parameter

The primary efficacy parameter is defined as the incidence rates of an ischemic event. The Cochran-Mantel-Haenszel Test will be used to compare the ischemic incidence rates between the 2 treatment groups. This test will provide us with a method of comparing the proportions between the 2 groups while adjusting for other variables in the model. Odds ratios with 95% confidence intervals will also be produced.

As a follow-up we will investigate the distribution of the first ischemic event (defined below) between the 2 treatment groups:

- Cardiac death,
- Cardiac arrest,
- Nonfatal MI,
- Ischemic CVA,

] - More Serious

- CABG,
- Recanalization procedure,
- Unstable angina (verified by objective evidence).

] - Less Serious

Descriptive statistics will be provided by treatment group on the total number of ischemic events.

**Analysis of the Secondary Efficacy Parameters**

The following secondary parameters will be analyzed:

- Time from randomization to the occurrence of an ischemic event;
- Change from baseline in angina class;
- Percent change from baseline to the end of treatment for the following lipid parameters:

  - LDL-C,
  - HDL-C,
  - Total Cholesterol,
  - Total TG,
  - Apo B,
  - Apo AI, and
  - Lp(a)

- QOL Measure; and
- All-Cause Mortality Rates.

In order to compare the 2 treatment groups on the time to the occurrence of an ischemic event, we will conduct a survival analysis using a Cox regression analysis on a model having the following structure:

$$\text{Time To Ischemic Event} = I_1\ I_2\ \text{Trial Treatment;}$$

where:

$I_1$ identifies the patient as having single or double vessel disease;
$I_2$ identifies the patient as asymptomatic or symptomatic;
Trial is an indicator variable that identifies the particular center associated with a patient; and
Treatment identifies the group to which the patient was randomized.

Using the Cox regression analysis, we will compare the 2 treatment groups on the survival times, while adjusting for the concomitant variables included in the model and taking into consideration any censored observations. The survival times for the censored observations will be defined as:

- If a patient withdraws before the 18-month period of follow-up, we will make every attempt to contact the patient in order to obtain the actual time an ischemic event occurred (if any); and if we are not successful in contacting the patient, the survival time will be the time period from randomization to the last contact period; and
- If an ischemic event does not occur during the study period, then the survival time will be the time period from randomization to the end of the study.

In addition, the trial by treatment interaction will be investigated.

Using PL (Product-Limit) Estimates given by Kaplan and Meir, we will provide estimates of the survival curves with 95% confidence intervals overall and within each center. In addition, we will provide graphs of the estimated survival curves for the 2 treatments.

The change from baseline in angina class, the percent change from baseline to the end of treatment associated with the lipid parameters described above, and the QOL measures will be analyzed using the following model:

$$Y = Y_B\ I_1\ I_2 \text{ Trial Treatment;}$$

where: $I_1$ and $I_2$ are defined above; and $Y_B$ corresponds to the baseline value of Y.

Ninety-five percent confidence intervals for the difference in the percent change for each of the parameters, overall and within each trial, will be generated. Again, we will investigate the trial by treatment interaction.

To compare the all-cause mortality rates between the 2 treatment groups, we will employ the Cochran-Mantel-Haenszel Test mentioned above in the discussion of the primary efficacy parameter. Again, odds ratios with 95% confidence intervals will be produced.

Other parameters of interest include:

- Patients in the atorvastatin group who undergo a PTCA or other recanalization procedure will have their subsequent ischemic events incidence rates compared with patients in the recanalization procedure/usual care group (all the patients in this group have a recanalization procedure);

- An evaluation of the number of PTCAs or other recanalization procedures required in the atorvastatin group compared to Group 2;

- A correlation analysis between the incidence rates of the ischemic events and the LDL-C and triglyceride measurements.

Also, an additional analysis of the primary efficacy parameter will be performed that will exclude those CABGs and recanalization procedures that occurred in either treatment group and were not indicated based on the criteria for intervention outlined in the Open-Label Treatment section.

Results of the foregoing study establishes that aggressive lowering of LDL with a cholesterol lowering agent is effective to prevent or delay the need for catheter-based revascularization. In the trial of 341 patients, only 13% of those who were treated with high doses (80 mg) of atorvastatin encountered an adverse heart event over the next 18 months, whereas 21% of the patients whose blockages were cleared with an angioplasty procedure suffered such adverse events within the same time period. The atorvastatin-treated patients had their LDL cholesterol reduced on average to 77 mg/dL from 140 mg/dL before treatment. That is well below the 130 mg/dL level considered as the target for people without heart disease, and the 100 mg/dL threshold for patients with symptoms of heart disease. LDL cholesterol in patients treated with angioplasty fell to about 119 mg/dL during the study. This study clearly establishes that LDL levels well below the 100 mg/dL goal is unexpectedly better and to be preferred. The results establish that aggressively lowering LDL by administering an effective amount of a cholesterol lowering agent will enable patients to forego costly angioplasty without fear of increasing their risk of a heart attack. The data establish that 87% of patients randomized to atorvastatin treatment, who were originally candidates for angioplasty, were instead able to remain on drug therapy alone for the duration of the 18-month trial, without experiencing any adverse cardiovascular events.

## Claims

1. Use of a cholesterol lowering agent in an amount to cause an aggressive lowering of LDL cholesterol by at least forty percent from'baseline for the preparation of a pharmaceutical composition preventing or delaying catheter-based revascularization in patients suffering from coronary artery disease and in need of such treatment.

2. Use according to claim 1 wherein the cholesterol is lowered to a level below 100 mg/dL.

3. Use according to Claim 1 wherein the cholesterol lowering agent is an HMG-CoA reductase inhibitor.

4. Use according to Claim 3 wherein the HMG-CoA reductase inhibitor is selected from atorvastatin, mevastatin, cerivastatin, simvastatin. fluvastatin, daivastatin, pravastatin, and lovastatin, or a pharmaceutically acceptable salt thereof.

5. Use according to Claim 4 wherein the HMG-CoA reductase inhibitor administered is atorvastatin, or a pharmaceutically acceptable salt thereof.

6. Use according to Claim 5 wherein the amount of atorvastatin administered is from 50 mg/day to 150 mg/day.

7. Use according to Claim 6 wherein atorvastatin is administered at a dose of about 80 mg/day.

8. Use according to Claim 1 wherein the cholesterol lowering agent is a carboxyalkyl ether of the formula

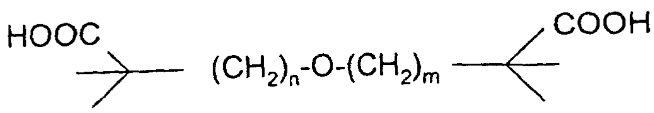

or a pharmaceutically acceptable salt thereof.

9. Use according to Claim 8 wherein the cholesterol lowering agent administered is 6,6'-oxybis-(2,2-dimethylhexanoic acid), or a pharmaceutically acceptable salt thereof

10. Use according to Claim 10 wherein the cholesterol lowering agent is selected from a fibrate.

11. Use according to Claim 9 wherein the cholesterol lowering agent is selected from clofibrate, gemfibrozil, fenofibrate, ciprofibrate, and benafibrate.

**Patentansprüche**

1. Verwendung eines cholesterinsenkenden Wirkstoffs in einer Menge, die eine dramatische Senkung von LDL Cholesterin um zumindest 40 % der Grundlinie verursacht, zur Herstellung einer pharmazeutischen Zusammensetzung, die eine auf Kathetern basierende Revaskularisation bei Patienten, die an koronaler Arterienerkrankung leiden und bei denen eine solche Behandlung notwendig wäre, vermeidet oder aufschiebt.

2. Verwendung nach Anspruch 1, worin der Cholesterinwert auf 10 einen Spiegel unter 100 mg/dL gesenkt wird.

3. Verwendung nach Anspruch 1, worin der cholesterinsenkende Wirkstoff ein HMG-CoA Reduktase-Hemmstoff ist.

4. Verwendung nach Anspruch 3, worin der HMG-CoA Reduktase-Hemmstoff aus den Stoffen Atorvastatin, Mevastatin, Cerivastatin, Simvastatin, Fluvastatin, Dalvastatin, Pravastatin, und Lovastatin, oder aus einem pharmazeutisch zulässigen Salz von diesen gewählt ist.

5. Verwendung nach Anspruch 4, worin der verabreichte HMG-CoA Reduktase-Hemmstoff Atorvastatin oder ein pharmazeutisch zulässiges Salz davon ist.

6. Verwendung nach Anspruch 5, worin die verabreichte Menge 25 von Atorvastatin zwischen 50 mg/Tag bis 150 mg/Tag beträgt.

7. Verwendung nach Anspruch 6, worin Atorvastatin in einer Dosis von etwa 80 mg/Tag verabreicht wird.

8. Verwendung nach Anspruch 1, worin der cholesterinsenkende Wirkstoff ein Carboxyalkyl Ether der Formel

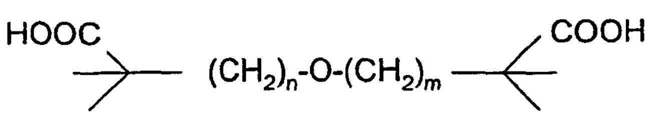

oder ein pharmazeutisch zulässiges Salz davon ist.

**9.** Verwendung nach Anspruch 8, worin der verabreichte cholesterinsenkende Wirkstoff eine 6,6'-Oxybis-(2,2-Dimethyl-Capronsäure), oder ein pharmazeutisch zulässiges Salz davon ist.

**10.** Verwendung nach Anspruch 1, worin der cholesterinsenkende Wirkstoff aus Fibraten ausgewählt ist.

**11.** Verwendung nach Anspruch 10, worin der cholesterinsenkende Wirkstoff aus Clofibraten, Gemfibrozil, Fenofibraten, Ciprofibraten und Benafibraten ausgewählt ist.

## Revendications

**1.** Utilisation d'un agent hypocholestérolémiant en une quantité permettant de provoquer une forte baisse du LDL-cholestérol d'au moins 40 % de la valeur initiale, pour la préparation d'une composition pharmaceutique prévenant ou retardant la revascularisation par cathéter chez des patients souffrant de coronaropathie et nécessitant un tel traitement.

**2.** Utilisation selon la revendication 1, dans laquelle le cholestérol est abaissé à un taux inférieur à 100 mg/dl.

**3.** Utilisation selon la revendication 1, dans laquelle l'agent hypocholestérolémiant est un inhibiteur de HMG-CoA réductase.

**4.** Utilisation selon la revendication 3, dans laquelle l'inhibiteur de HMG-CoA réductase est choisi parmi l'atorvastatine, la mévastatine, la cérivastatine, la simvastatine, la fluvastatine, la dalvastatine, la pravastatine et la lovastatine ou un sel pharmaceutiquement acceptable d'un tel composé.

**5.** Utilisation selon la revendication 4, dans laquelle l'inhibiteur de HMG-CoA réductase administré est l'atorvastatine ou un sel pharmaceutiquement acceptable de celle-ci.

**6.** Utilisation selon la revendication 5, dans laquelle la quantité d'atorvastatine administrée va de 50 mg/jour à 150 mg/jour.

**7.** Utilisation selon la revendication 6, dans laquelle l'atorvastatine est administrée à une dose d'environ 80 mg/jour.

**8.** Utilisation selon la revendication 1, dans laquelle l'agent hypocholestérolémiant est un éther carboxyalkylique de formule

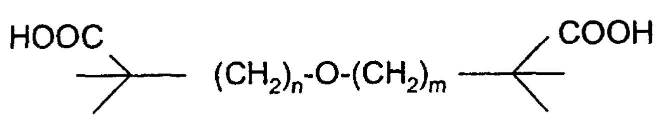

ou un sel pharmaceutiquement acceptable d'un tel composé.

**9.** Utilisation selon la revendication 8, dans laquelle l'agent hypocholestérolémiant est l'acide 6,6'-oxybis-(2,2-diméthylhexanoïque) ou un sel pharmaceutiquement acceptable de celui-ci.

**10.** Utilisation selon la revendication 1, dans laquelle l'agent hypocholestérolémiant est choisi parmi des fibrates.

**11.** Utilisation selon la revendication 10, dans laquelle l'agent hypocholestérolémiant est choisi parmi le clofibrate, le gemfibrozil, le fénofibrate, le ciprofibrate et le bénafibrate.